# EUROPEAN PATENT APPLICATION

(11) **EP 0 818 211 A1**
(43) Date of publication of application: **14.01.1998**
(21) Application number: 96305086.9
(22) Date of filing: 10.07.1996
(51) Int. Cl.: A61M 5/32, A61M 5/34, A61M 5/50, A61M 5/315

(54) **Simplified safety syringe with retractable self-biased needle and minimized plunger**

(71) Applicant: Chen, Long-Hsiung, Taipei (TW)
(72) Inventor: Chen, Long-Hsiung, Taipei (TW)
(74) Representative: Selby-Lowndes, Guy Francis Charles

(57) **Abstract**

A safety syringe comprises: a needle device (2) having a hollow needle (21) held fixedly in a insilient shank (22) formed a bias arrowhead socket (27) in a rear body of the shank (22), a syringe means (1) having a hollow cylinder (11) with a reduced sleeve (12) at a front for receiving the shank (22) therein, a plunger means (3) having an arrowhead (301) coupling member (30) disposed to a front center of a plunger head (31) thereon; the arrowhead (301) of the plunger head (31) will be sqeezed into the bias arrowhead socket (27) of the shank (22) to engage the needle device (2) and the plunger head (31) together while the plunger means (3) is fully pushed forwardly after an injection; and then the needle device (2) will be retracted fully into the cylinder (11) and biased off from a center line of the cylinder (11) while the plunger means (3) has been drawn back to imited position; after then by pushing the plunger means (3) forwardly again, the needle (21) will be sent and destroyed against a shoulder of the cylinder (11) and safely remained in the cylinder.

## Description

### BACKGROUND OF THE INVENTIONS

U.S. Pat. No.5402327 entitled "Simplified Safety Syringe With Retractable Self-Biased Needle " issued to the same inventor of this application disclosed a safety syringe including a hollow needle normally held in a front portion of the syringe, a coupling member of arrowhead shape integrally formed with a thin-disk plunger, an annular ring embedded on the plunger to allow the plunger to be slidably held in the syringe for injection use, the coupling member formed on the plunger engageable with a bias socket recessed in a rear portion of the hollow needle in which the biasing socket generally formed as a conical shape with a longitudinal center line biased against the center line of the needle, whereby upon retraction of the plunger and the coupled needle into the syringe, the needle will be biased to prevent a further outward protruding of the retracted needle.

However, the needle portion of the prior art has a heavy shank and an acute packing ring circumferentially disposed around the shank to be engageably held in a ring groove in a sleeve. Since the safety syringe is always made disposable and will be disposed once being used for, hygienic reason. This will waste money and will also increase a burden for waste disposal and treatment on a viewpoint of environmental protection because the shank is big and mostly made of resilient rubber material.

### SUMMARY OF THE INVENTION

A safety syringe comprising a syringe means, a needle device and a plunger means, in which:
Said syringe means includes a hollow cylinder for filing liquid medicine therein, and a reduced sleeve provided at a reduced shoulder on a front portion of said cylinder having a central opening formed a bottle-neck inside said sleeve, an outward flange and a inward stop ring provided to a rear edge of said cylinder thereon;
Said needle device includes a hollow needle having a needle tip at a front portion, a resilient rubber shank fixedly held a rear portion of said needle having an annular recess between a cylindrical rear body and a streamline front body so as to be held in said sleeve of said syringe means by said bottle neck therein, said cylindrical rear body of said shank having a bias rear end surface and a bias conical space formed of an arrowhead socket with a ratchet-means gate at a center of said bias rear end surface led to a hollow center of said hollow needle through said arrowhead socket therefrom;
Said plunger means is made of solid plastic comprising a plunger head having a annular ring groove and a plunger ring been received therein which slidably held in said cylinder of said syringe means to push liquid medicine for injection operation, an arerowhead coupling member forwardly disposed to a center of a front end of said plunger head having a ratchet-means neck at a root portion thereof which said arrowhead will be inserted into said bias arrowhead socket through said ratchet-means gate of said rear body of said shank and firmly engaged said needle device to said plunger means together as said ratchet-means neck of said arrowhead of siad plunger head been caught by said ratchet-means gate of said shank while said plunger means been fully pushed forward during finished an injection operation, furthermore said bias arrowhead socket of resilient material been distorted to a right status from a bias status by said arrowhead of solid material forced thereinto, simultaneously an insilient strain been stored in the resilient material of said rear body of said shank which caused said needle tip to be biased to said sleeve of said syringe means inside said cylinder while said needle device been fully pulled back into said cylinder by a retracting of said plunger means after an injection operation and then said needle will be bent and destroyed ad safely remain in said cylinder while said needle tip be blocked against said reducing shoulder of said cylinder during said plunger means been pushed again forwardly into said cylinder after a fully retracting of said plunger means; a thin disk provided to a plunger rod behind said plunger head to an appropriate distance for limiting a drawn back motion of said plunger means in contacting against to said stop ring of said cylinder thereof, a plunger rod formed of three blades protruding radially from a longitudinal center line of said plunger means disposed to a rear face of said plunger head and extended longitudinally out to said stop ring of said cylinder, and an end plate disposed to a rear end of said plunger rod for handling the pushing or pulling operation of said plunger means therefor.

### BRIEF DESCRIPTION OF THE DRAWINGS

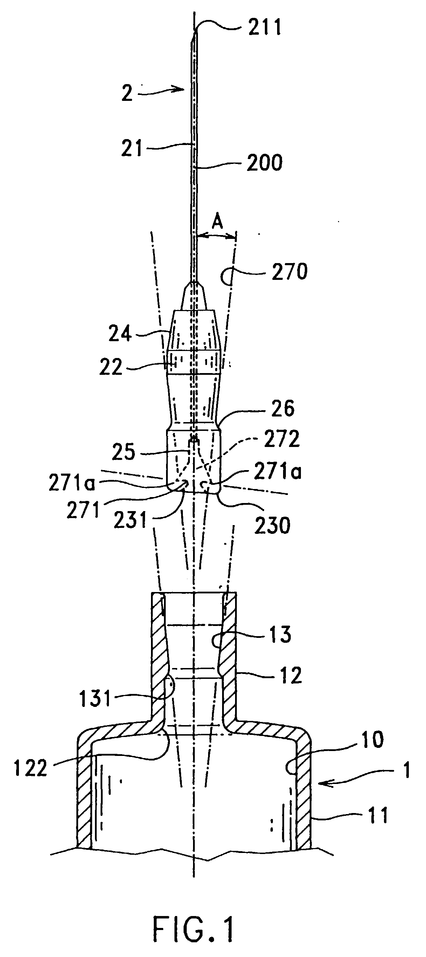

Fig. 1 is an illustration showing the syringe and the needle device in accordance with the present invention;
Fig. 2 is an illustration showing the present invention when serving for an injection operation according to the present invention;
Fig. 3 shows the coupling member arrowhead of the plunger head engaged with the arrowhead socket of the shank of the needle device when finishing a medical injection;
Fig. 4 shows the needle been retracted wholly into the cylinder of the present invention'
Fig. 5 shows the needle been destroyed ad remained in the cylinder by pushing the plunger forward again afier a fully retracting in accordance with the present invnetion;
Fig. 6 is an illustration showing the plunger of the present invention in a perspective view; and
Fig. 7 is an illustration showing the plunger rod with three blades according to the present invention in a sectional view.

### DETAILED DESCRIPTION

As shown in the figures, the present invnetion comprises a syringe means 1, a needle device 2, and a plunger means 3.

The syringe means 1 includes: a cylinder 11 having a hollow bore 10 for filling liquid medicine 4 therein, a reduced sleeve 12 formed on a front portion of the cylinder 11 extended forwardly from a shoulder 122 of the cylinder 11 having a central opening 13 formed a bottle neck 131 through the sleeve 12, a flange 14 formed radially outwardly on a rear end of the cylinder 11, and an annular stop ring 16 extended inwardly from the flange 14.

The needle device 2 includes: a needle 21 having a needle tip 211 at a front end thereof, a shank 22 which made of insilient rubber material connected with the needle 21 having a streamline front body 24, a cyindrical rear body 25 and an annular recess 26 therebetween so that the shank 22 could be held in the sleeve 12 by the bottle neck 131 thereof that the shank 22 has a bais rear end surface 230, a bias socket 27 of conical shape, formed in the rear body 25 of the shank 22 and communicating with a ratchet-means gate 231 from the rear end surface 230.

A needle center line 200 is aligned with a syringe center line 100 when the needle device 2 is normally secured in sleeve 12 of the syringe means 1 for injection purpose.

The biasing socket 27 is generally conically shaped and includes: a conical bottom 271 and conical apex 272 tapered forwardly from the conical bottom 271. A longitudinal conical axis 270 aligned with the conical apex 272 is generally perpendicular to the conical bottom 271 and to be inclindedly deviated from the needle axis 200 of the needle device 2 to define a acute angle A between the needle axis 200 and the longitudinal conical axis 270 of the biasing socket 27. The conical bottom 271 includes a ratchet-tooth recess 271a circumferentially recessed in a rear portion of the shank portion 22 and tapered radially rearwardly from the conical bottom 271 of the biasing socket 27. The biasing socket 27 is snugly engageable with an arrowhead portion 301 of the plunger means 3 for obliquely biasing the needle device 2 when coupled to the plunger means 3 and retracted into the syringe cylinder 11 after finishing an injection.

The plunger means 3, which is made of solid plastic material, includes a plunger head 31, a coupling member 30, and a plunger rod 35. Plunger head 31 having an annular ring groove 311 for accommodating an annular plunger ring 38 for ensuring a smooth sealable sliding of the plunger head 31 in the cylinder 11. The annular plunger ring 38 has a cross section of circular shape in this embodiment but it can be of other shape. The coupling member 30 has an arrowhead 301 with a ratchet-means neck 302 is located at a front end of the plunger head 31 which can be inserted into the bias socket 27 of the shank 22 through the ratchet-means gate 231. The plunger rod 35 is formed of three blades 351 protruding radially from a longitudinal center line 300 of the plunger means 3. The plunger rod 35 is secured with an end plate 37 at an end edge thereof for facilitating the pushing and retracting operation of the plunger means 3 and a thin disk 39 which is radially outwardly attached to the plunger rod 35.

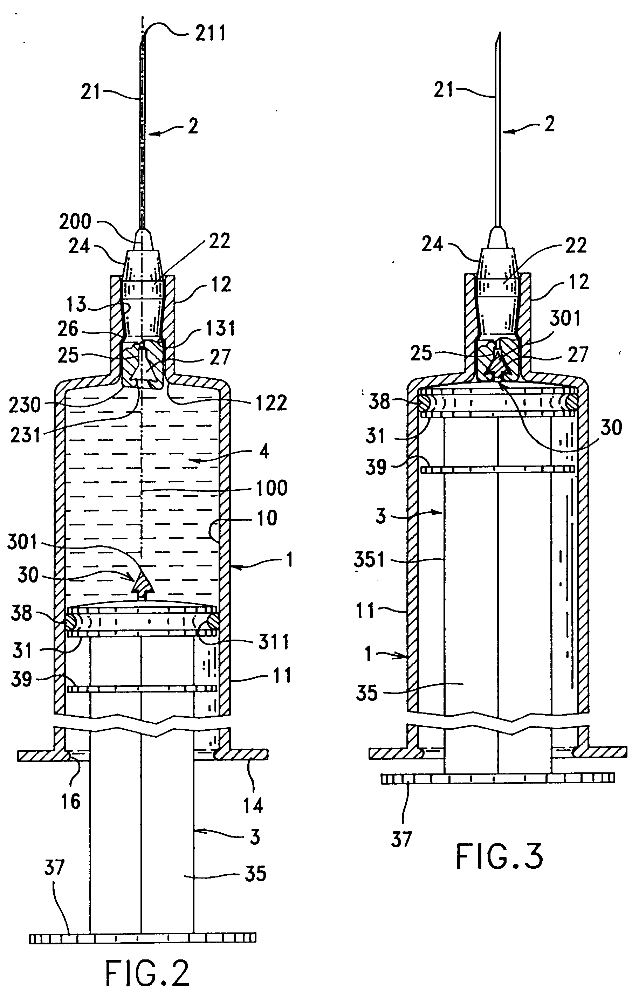

When using the present invention for injection as shown in Fig. 2, the plunger means 3 is pushed forwardly so that the medicine 4 in the cylinder 11 can go through the needle device 2 into a patient's body.

When the plunger means 3 has been fully pushed forwardly after a injection, as shown in Fig. 3. The arrowhead portion 301 of the coupling member 30 will be inserted into the bias socket 27 of the needle device 2 to make a firm engagement of the plunger means 3 with the needle device 2, and forcibly distort the rear body 25 of the shank 22, to a right status from a biased status and stored a resilient potential energy therein.

During a retracting of the plunger means 3, after an injection operation, the needle device 2 will be then drawn backwardly with the plunger means 3. It is easy to pull the shank 22 backwardly through the bottle neck 131 of the sleeve 12 because the front body 24 of the shank 22 is of a streamlined-shape.

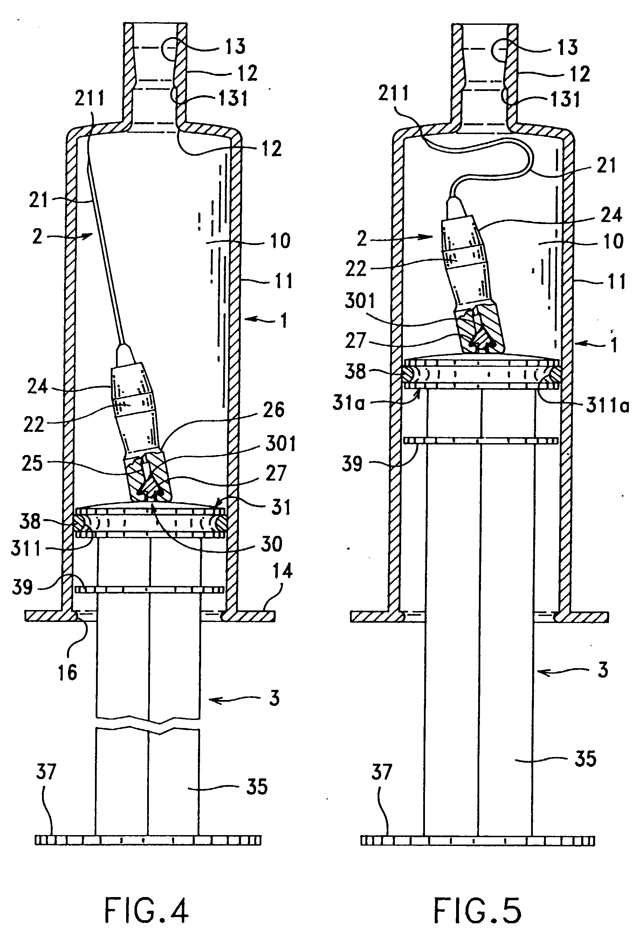

After retracting, the plunger head 31 pulled the neddle device 2 wholly into the bore portion 10 of the cylinder 11 as shown in Fig. 4, the needle device 2 will be automatically biased off from the syringe center line 100 by the resilient potential energy since the bias socket 27 of the shank 22 is released from the sleeve 12 the arrowhead portion 301 of the coupling member 30 of the plunger means 3 by releasing a resilient force stored in the distorted rear body 25 of the shank 22.

After re-protruding the needle device 2 outwardly as shown in Fig. 5, the needle tip 211 will be pushed against the reducing shoulder 122 formed in a front portion of the cylinder 11, thereby the needle 21 is bent and its outward protrusion is safely obstructed to prevent it from injuring the people.

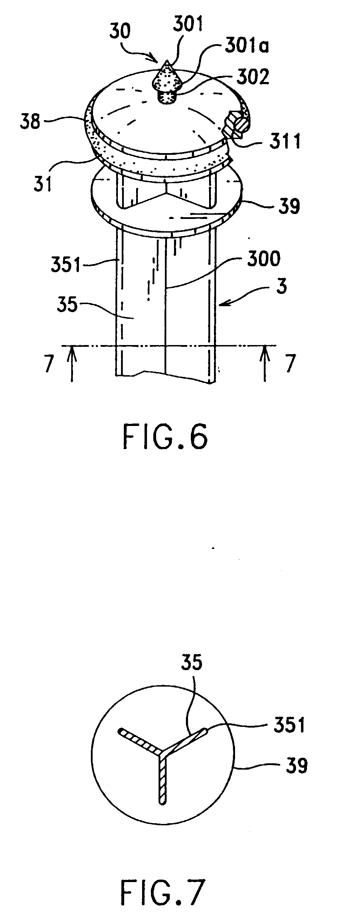

The present invention is superior to the earlier invention U.S. Pat. No.5402327 issued to the same inventor of this application since the shank portion 22 of needle device 2 has been formed of a streamline front body 24 and the plunger rod 35 of the earlier invention has been minimized to merely of three blades (referring to Fig. 7), thereby saving cost and reducing the secondary pollution of environment.

The plunger means 3 in a preferred embodiment is shown in Fig. 6, in which a thin disk 39 is located at a front portion of the plunger rod 35 behind the plunger head 31, and been set and kept an appropriate distance from the plunger head 31 to limit the backward stroke by an abutment of the thin disk 39 with the stop ring 16 of the cylinder 11 for ensuring enough space to allow the needle device 2 to be fully retracted back into the cylinder 11.

## Claims

1. A safety syringe comprising a syringe means, a needle device and a plunger means, in which:
said syringe means includes a hollow cylinder for filing liquid medicine therein, and a reduced sleeve provided at a reduced shoulder on a front portion of said cylinder having a central opening formed a bottle-neck inside said sleeve, an outward flange and a inward stop ring provided to a rear edge of said cylinder thereon;
said needle device includes a hollow needle having a needle tip at a front portion, a resilient rubber shank fixedly held a rear portion of said needle having an annular recess between a cylindrical rear body and a streamline front body so as to be held in said sleeve of said syringe means by said bottle neck therein, said cylindrical rear body of said shank having a bias rear end surface and a bias conical space formed of an arrowhead socket with a ratchet-means gate at a center of said bias rear end surface led to a hollow center of said hollow needle through said arrowhead socket therefrom;
said plunger means is made of solid plastic comprising a plunger head having a annular ring groove and a plunger ring been received therein which slidably held in said cylinder of said syringe means to push liquid medicine for injection operation, an arrowhead coupling member forwardly disposed to a center of a front end of said plunger head having a ratchet-means neck at a root portion thereof which said arrowhead will be inserted into said bias arrowhead socket through said ratchet-means gate of said rear body of said shank and firmly engaged said needle device to said plunger means together as said ratchet-means neck of said arrowhead of said plunger had been caught by said ratchet-means gate of said shank while said plunger means been fully pushed forward during finished an injection operation, furthermore said bias arrowhead socket of resilient material been distorted to a right status from a bias status by said arrowhead of solid material forced therinto, simultaneously an insilient strain been stored in the resilient material of said rear body of said shank which cased said needle tip to be biased to said sleeve of said syringe means inside said cylinder while said needle device been fully pulled back into said cylinder by a retracting of said plunger means after an injection operation and then said needle will be bent and destroyed and safely remain in said cylinder while said needle tip be blocked against said reducing shoulder of said cylinder during said plunger means been pushed again forwardly into said cylinder after a fully retracting of said plunger means; a thin disk provided to a plunger rod behind said plunger head to an appropriate distance for limiting a drawn back motion of said plunger means in contacting against to said stop ring of said cylinder thereof, a plunger rod formed of three blades protruding radially from a longitudinal center line of said plunger means disposed to a rear face of said plunger head and extended longitudinally out to said stop ring of said cylinder, and an end plate disposed to a rear end of said plunger rod for handling the pushing or pulling operation of said plunger means therefor.

2. A safety syringe as claimed in claim 1 wherein said plunger rod is formed of three blades radially from a longitudinal center line of said plunger means.

3. A safety syringe as claimed in claim 1 wherein said thin disk provided to said plunger rod has been set an appropriate distance from said plunger head to provide enough space for retracting whole said needle fully into said cylinder and to prevent said plunger means from being pulled off from said cylinder because of an abutment of said thin disk with said stop ring of said cylinder during a pulling back motion of said plunger means.
